# EUROPEAN PATENT APPLICATION

(11) **EP 4 358 089 A1**
(43) Date of publication of application: **24.04.2024**
(21) Application number: 22202309.5
(22) Date of filing: 18.10.2022
(51) Int. Cl.: G16H 20/13, A61J 1/03, A61J 7/00, G06K 7/14

(54) **A SYSTEM AND METHOD OF ASSEMBLING A DRUG MEDICAMENT TRAY**

(71) Applicant: Medistac Inc. Limited, 1 Dublin (IE)
(72) Inventor: Beggs, David, Dublin, D03 PW57 (IE); Walkin, Kieran, Dublin, A96 Y276 (IE)
(74) Representative: Purdylucey Intellectual Property

(57) **Abstract**

Described is a system and method to assemble a medicament dispensing tray comprising a tray housing having a machine-readable tray housing identification code and a plurality of drug blister strips mounted to receptacles of the tray housing, each strip having a machine-readable strip identification code. The system typically comprises a holder to hold the tray housing while drug blister strips are mounted to the tray housing and comprising a holder housing configured to receive the tray housing, a first sensor configured to detect a tray housing identification code of the tray housing received within the holder housing, and a plurality of second sensors each configured to detect a strip identification code of the drug blister strip mounted to the tray housing received within the housing, and software executable on a computational device comprising program instructions configured to instruct an assembly operator on how to assemble the dispensing tray according to a patients prescription, to check that the prescription has been filled correctly, and/or to retain a retrievable record of the patient specific dispensing tray.

## Description

### Technical Field

The present invention relates to a system and method of assembling a medicament dispensing tray. Also described is a holder for use in assembling a medicament dispensing tray.

### Background

Currently, the assembly of dispensing drugs in a pharmacy involves a pharmacist counting out the required number of tablets for each drug for a given period such as a month and providing the drugs to the patient. This is time consuming and laborious, especially when a patient is taking a number of different medications. In the case of older patients taking a number of medications, the tablets are often provided in drug dispensing trays that have multiple individual containers for tablets, for example a weekly tray having a container for each day of the week or a monthly container having a container for each day of the month. Each container may contain multiple tablets, including tablets that are to be taken in the morning and tablets that are to be taken at a different time. Preparation of this type of dispensing tray is time consuming and laborious, especially as tablets that are provided in a blister pack form have to be removed from blisters.

Some pharmacies provide weekly or monthly medication for patients in the form of large tailor-made blister packs, with the tablets for each day of the week or month provided in an individual blister chamber. These blister packs are time consuming and laborious to prepare and add a further cost to the price of obtaining medication. In addition, if a dispensing tray or blister pack is filled incorrectly, this can have serious consequences for the patient, and also for the pharmacy in terms of professional liability. It is therefore an object of the invention
reduce the risk of incorrect medication being filled during the filling process. A further object of the invention is to remove the need for technicians or pharmacists to handle the tablets. A further object of the invention is to dramatically increase the efficiency of the filling process. A further object of the invention is to introduce traceability from individual batches of medication back to individual clients.

### Summary

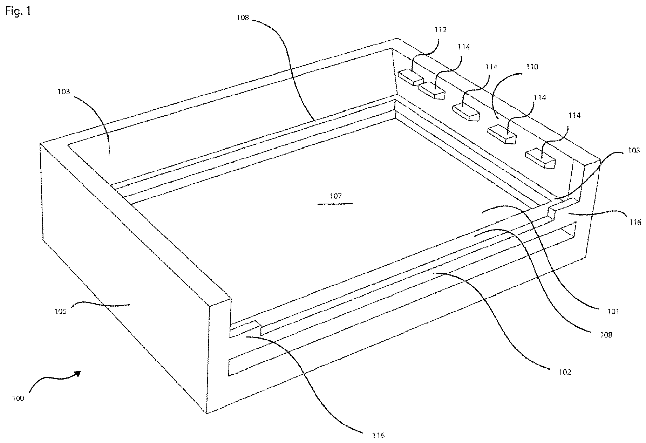

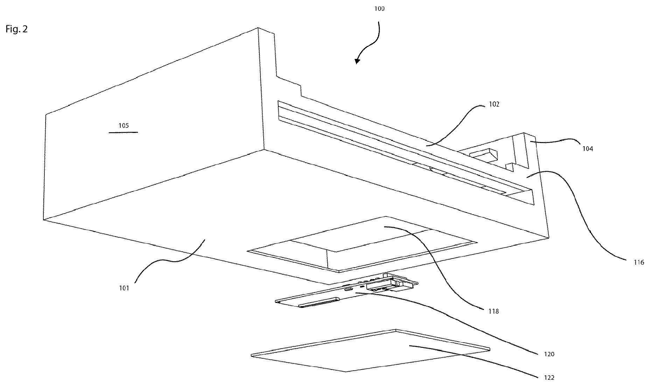

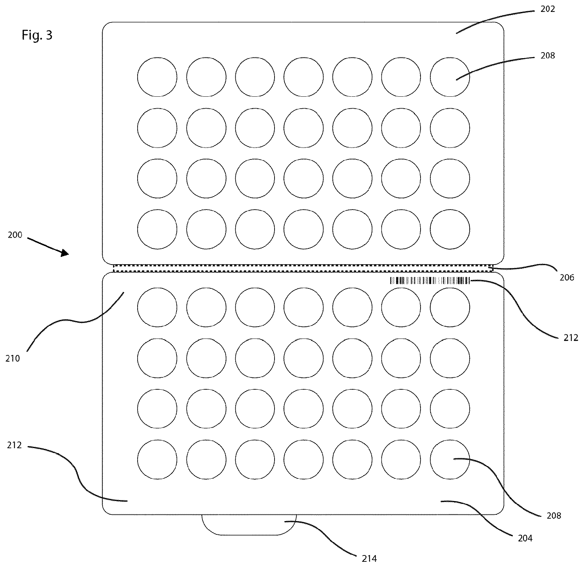

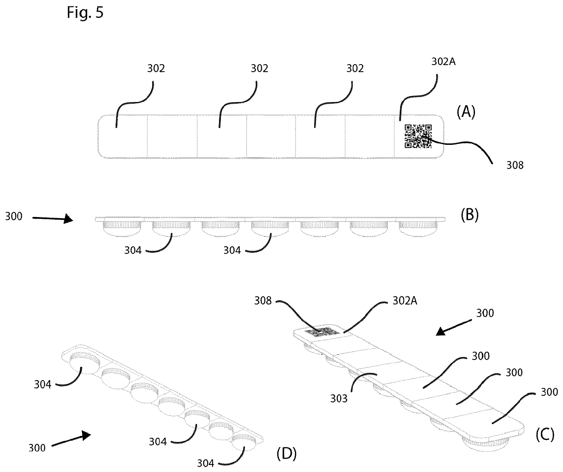

The objective is met by the provision of a system and method of assembling a medicament dispensing tray. The dispensing tray comprises a tray housing configured to receive and hold multiple drug blister strips, one embodiment of which is illustrated in Figure 5. The tray housing may take the form of a booklet shown in Figure 3 having a front and read page with aligned holes dimensioned to receive the drug blister strips. The system preferably also includes a holder to hold the tray housing while drug blister strips are mounted to the housing (See Figs 1 and 2). The holder has a first sensor to detect and record a unique identity of a tray housing, and unique identities of drug blister strips mounted in the tray housing. The system includes software that is executable on a computation device such as a computer or mobile communications device, to instruct an assembly operator on how to assemble the dispensing tray according to a patient's prescription, to check that the prescription has been filled correctly, and to retain a retrievable record of the patient specific dispensing tray.

In a first aspect, the invention provides a system to assemble a medicament dispensing tray of the type comprising a tray housing having a machine-readable tray housing identification code and a plurality of drug blister strips mounted to receptacles of the tray housing, each drug blister strip having a machine-readable strip identification code (308), the system comprising:
a device comprising a sensor system configured to detect a tray housing identification code and strip identification codes; and
software executable on a computational device comprising program instructions configured to:
   retrieve, by the computational device from a patient database stored on a memory, patient data comprising patient identification data and drug prescription data for the patient;
display the retrieved drug prescription data on a graphical display;
   detect, by the sensor system a tray housing identification code of a tray housing;
   detect, by the sensor system, strip identification codes of drug blister strips mounted to the tray housing by the assembly operator;
   compare the drug prescription data with the detected strip identification codes;
   display a message on the graphical display based on the comparison;
   compile a patient-specific data package for the patient comprising the patient identification data, tray identification code, and the plurality of strip identification codes; and
   store, by a memory, the patient-specific data package.

In any embodiment, the software comprises program instructions configured to:
cause the graphical display to display details of a first drug of the drug prescription on the display;
detect with the sensor system when a drug blister strip corresponding to the first drug is mounted to the tray housing;
optionally, display on the graphical display a message indicating that the first drug was successfully added to the tray housing;
display details of a second drug of the drug prescription on the display;
detect with the sensor system when a drug blister strip corresponding to the second drug is mounted to the tray housing; and
optionally, displaying on the graphical display a message indicating that the second drug was successfully added to the tray housing;

In any embodiment, the device comprising a sensor system is a mobile communications device with a camera and software for reading machine readable codes.

In any embodiment, the system comprises a holder to hold the tray housing while drug blister strips are mounted to the tray housing, the holder comprising a holder housing configured to receive the tray housing, and the sensor system.

In any embodiment, the sensor system comprises a first sensor configured to detect a tray housing identification code of the tray housing received within the holder housing, and one or more second sensors each configured to detect a strip identification code of the drug blister strip mounted to the tray housing received within the housing.

In any embodiment, the processor comprises program instructions configured to cause the graphical display to display an error message when a second sensor detects a strip identification code corresponding to a drug that does not form part of the drug prescription data.

In any embodiment, the processor comprises program instructions configured to cause a printer coupled to the computational device to print the patient-specific data package on to a medium that is attachable to the tray housing.

In any embodiment, the patient-specific data is printed in the form of a bar code or QR code.

In any embodiment, the patient-specific data package comprises identification of the assembly operator.

In any embodiment, the patient identification is anonymised.

In any embodiment, the software comprises program instructions configured to cause the graphical display to display for the assembly operator (a) instructions comprising mounting specific drug blister strip to specific receptables of the tray housing and (b) an indication when the instructions have been successfully carried out.

In any embodiment, the software comprises program instructions configured to cause the graphical display to display an indication when all the drug blister strips have been mounted to the tray housing correctly.

In any embodiment, the patient-specific data package for the patient comprises data relating to the batch/lot of the drugs contained in one or more of the drug blister strips of the medicament dispensing tray.

In any embodiment, the patient-specific data package for the patient comprises information relating to how the drugs in the drug blister strips of the medicament dispensing tray relates back to an original prescription.

In any embodiment, the patient-specific data package for the patient comprises the scheduled and/or actual delivery date of the medicament dispensing tray to the patient.

In any embodiment, the tray housing identification code and the strip identification codes are each, independently, selected from bar codes and QR codes.

In any embodiment, the tray housing comprises a booklet comprising a front page and a back page, the front page having an array of apertures comprising at least two rows of apertures, in which the back page comprises a corresponding array of apertures configured to register with the apertures of the front page when the booklet is closed.

In any embodiment, the plurality of drug blister strips includes a first blister strip containing a first drug and a second blister strip containing a second drug, wherein each blister strip comprises a row of blisters corresponding to the plurality of apertures in each row of apertures allowing the blister strip to nest snugly in a row of apertures of the front or back page of the booklet.

In any embodiment, each row of the booklet comprises N apertures and the blister strips each include a row of N blisters. N can be any whole number >1, for example 7, 14, 21 or 28. The booklet generally has 2-10 rows.

In any embodiment, the front page and back page are hingedly connected together.

In any embodiment, the software comprises program instructions configured to cause the graphical display to display instructions for the assembly operator to seal the booklet in a closed configuration when the detected strip identification codes correspond to the drug prescription data.

In any embodiment, the system includes a graphical display, that in one embodiment may be attached to the holder.

In any embodiment, the computational device is selected from a laptop or desktop computer, a tablet, or a mobile communications device.

In any embodiment, the holder comprises a wireless communications module configured for wireless communication with the computational device.

In any embodiment, the holder comprises a holder of the invention (as set out below).

**In a second aspect,** the invention provides a method of assembling a medicament dispensing tray comprising a tray housing having a machine-readable tray housing identification code and a plurality of drug blister strips mounted to receptacles of the tray housing, each strip having a machine-readable strip identification code,

The method typically employs a holder to hold the tray housing while drug blister strips are mounted to the tray housing. The holder generally comprises a holder housing configured to receive the tray housing, a first sensor configured to detect a tray housing identification code of the tray housing received within the holder housing, and one or more (generally a plurality of) second sensors each configured to detect a strip identification code of the drug blister strip mounted to the tray housing received within the housing.

In any embodiment, the method comprises the steps of:
retrieving from a patient database stored on a memory, patient data comprising patient identification data and drug prescription data for the patient;
displaying the retrieved drug prescription data on a graphical display;
placing a tray housing in the holder housing;
detecting, by a first sensor, a tray identification code of the tray housing (that is optionally placed within the holder);
mounting a plurality of drug blister strips to receptacles of the tray housing according to the retrieved drug prescription data displayed on the graphical display;
detecting, by a plurality of second sensors, strip identification codes of drug blister strips mounted to the dispensing tray;
comparing the retrieved drug prescription data with the detected strip identification codes;
displaying a message on the graphical display based on the comparison;
compiling a patient-specific data package for the patient comprising the patient identification data, tray identification code, and the plurality of strip identification codes; and
storing, by the memory, the patient-specific data package.

The holder comprising a first and second sensor may be employed as part of the method. In another embodiment, a holder is not required in which case another device with a sensor such as a camera, for example a mobile communications device, may be employed to read the codes on the tray housing and the codes on each blister strip. Thus, the first and second sensors may be provided by a mobile communications device with a camera and software configured to read machine readable codes. The software is generally configured to cause the mobile device to read the codes using the mobile device camera and perform the other steps of the method.

In any embodiment, the method comprises the steps of:
displaying details of a first drug of the retrieved drug prescription on the graphical display;
mounting a drug blister strip containing the first drug to a receptable of the tray housing;
detecting with a second sensor when the drug blister strip containing the first drug is mounted to the tray housing;
optionally, displaying on the graphical display a message indicating that the first drug was successfully added to the tray housing;
displaying details of a second drug of the drug prescription on the graphical display; and
mounting a drug blister strip containing the second drug to a receptable of the tray housing; and
detecting with a second sensor when the drug blister strip containing the second drug is mounted to the tray housing;
optionally, displaying on the graphical display a message indicating that the second drug was successfully added to the tray housing;

In any embodiment, the method includes a step of displaying an error message on the graphical display when a second sensor detects a strip identification code corresponding to a drug that does not form part of the drug prescription data.

In any embodiment, the method includes a step of printing the patient-specific data package on to a medium that is attachable to the tray housing.

In any embodiment, the patient-specific data is printed in the form of a bar code or QR code.

In any embodiment, the patient-specific data package comprises identification of the assembly operator.

In any embodiment, the patient identification is anonymised.

In any embodiment, the method includes a step of displaying on the graphical display instructions comprising mounting specific drug blister strip to specific receptables of the tray housing and an indication when the instructions have been successfully carried out.

In any embodiment, the method includes a step of displaying on a graphical display an indication when all the drug blister strips corresponding to the drug prescription have been mounted to the tray housing correctly.

In any embodiment, the patient-specific data package for the patient comprises data relating to the batch/lot of the drugs contained in one or more of the drug blister strips of the medicament dispensing tray.

In any embodiment, the patient-specific data package for the patient comprises information relating to how the drugs in the drug blister strips of the medicament dispensing tray relates back to an original prescription.

In any embodiment, the patient-specific data package for the patient comprises the scheduled delivery date of the medicament dispensing tray to the patient.

In any embodiment, the tray housing identification code and the strip identification codes are each, independently, selected from bar codes and QR codes.

In any embodiment, the tray housing comprises a booklet comprising a front page and a back page, the front page having an array of apertures comprising at least two rows of seven apertures, in which the back page comprises a corresponding array of apertures configured to register with the apertures of the front page when the booklet is closed.

In any embodiment, the plurality of drug blister strips includes a first blister strip containing a first drug and a second blister strip containing a second drug, wherein each blister strip comprises a row of seven blisters corresponding to the plurality of apertures in each row of apertures allowing the blister strip to nest snugly in a row of apertures of the front or back page of the booklet.

In any embodiment, the front page and back page are hingedly connected together.

In any embodiment, the method includes a step of displaying on the graphical display instructions for the assembly operator to seal the booklet in a closed configuration when the detected strip identification codes correspond to the drug prescription data.

In any embodiment, the computational device is selected from a laptop or desktop computer, a tablet, or a mobile communications device.

In any embodiment, the holder comprises a wireless communications module configured for wireless communication with the computational device.

**In a third aspect,** the invention provides a holder to hold a tray housing of a medicament dispensing tray while drug blister strips are mounted to the tray housing, the holder comprising:
a holder housing configured to receive the tray housing:
a first sensor configured to detect a tray housing identification code of the tray housing received within the holder housing; and
a plurality of second sensors each configured to detect a strip identification code of the drug blister strip mounted to the tray housing received within the housing.

In any embodiment, the holder has a top surface comprising a recessed part dimensioned to receive the tray housing (or a front or rear page when the tray housing has a booklet format).

In any embodiment, the recessed part is defined by a sidewall, wherein the first and second sensors are mounted to the sidewall.

In any embodiment, the sidewall has a front wall, rear wall, and opposed side walls, in which the first and second sensors are attached to one of the side walls.

In any embodiment, the front wall tapers outwardly.

In any embodiment the first and/or second sensors are barcode or QR code scanners.

Other aspects and preferred embodiments of the invention are defined and described in the other claims set out below.

### Brief Description of the Figures

**FIG. 1** is a perspective view of a tray holder of the invention.
**FIG. 2** is a perspective, partly exploded view, on the underside of the tray holder of Figure 1.
**FIG. 3** is a top plan view of a tray housing forming part of a medicament dispensing tray and having four rows of seven apertures (each row forms a receptable for a drug blister strip shown in Figure 5).
**FIG. 4** is a perspective view showing the tray housing nested in the tray holder.
**FIG. 5** shows a drug blister strip forming part of a medicament dispensing tray including a top plan view (A), side elevational view (B), perspective view from above (C) and perspective view from below (D).
**FIG. 6** is a perspective view of the tray housing nested in the tray holder and four drug blister strips mounted in the receptables of the tray housing,
**FIG. 7** is a perspective view of a graphical display forming part of the system of the invention.
**FIG. 8** is a block diagram illustrating the method of the invention.
**FIG. 9** is a perspective view of the tray housing nested in an alternative tray holder and four drug blister strips mounted in the receptables of the tray housing. In this embodiment, the tray holder has a single barcode reader mounted on an arm and configured to read the barcodes of all four blister strips.

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g., a feature, element, characteristic, property, method/process step or limitation) or group of integers (e.g., features, element, characteristics, properties, method/process steps or limitations) but not the exclusion of any other integer or group of integers. Thus, as used herein the term "comprising" is inclusive or openended and does not exclude additional, unrecited integers or method/process steps.

The embodiments in the invention described with reference to the drawings comprise a computer apparatus and/or processes performed in a computer apparatus. However, the invention also extends to computer programs, particularly computer programs stored on or in a carrier adapted to bring the invention into practice. The program may be in the form of source code, object code, or a code intermediate source and object code, such as in partially compiled form or in any other form suitable for use in the implementation of the method according to the invention. The carrier may comprise a storage medium such as ROM, e.g., CD ROM, or magnetic recording medium, e.g., a floppy disk or hard disk. The carrier may be an electrical or optical signal which may be transmitted via an electrical or an optical cable or by radio or other means.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

Referring to the drawings and initially to Figures 1 and 2, there is illustrated a holder of the invention, indicated generally by the reference numeral 100. The holder comprises a rectangular housing having a base 101, front wall 102, rear wall 103, and side walls 104 and 105. The top comprises a recessed surface 107 and a shoulder 108 that extends around the front, rear and side walls above the recessed surface 107. The real wall 103 and side walls 104, 105 have the same height, whereas the front wall 102 is shallower providing an opening for a tray housing to be inserted into the holder and nested on the shoulder 108. The rear wall 103 is slanted outwardly as illustrated in Figure 1. An inner surface 110 of the sidewall 104 has a number of sensors mounted hereto, namely a first sensor in the form of a barcode reader 112 and four second sensors in the form of barcode readers 114. The sensors are mounted to the sidewall towards a top of the sidewall and spaced from the base, with the reader part of the sensors facing downwardly towards the recessed base 101. The front wall 102 also includes corner stop sections 116 to keep a tray housing in place on the recessed base.

Referring to Figure 2, the base 101 of the holder includes a recess 118 for housing a processor and wireless communications module 120 and a lid 122 for the recess.

Referring to Figure 3, a tray housing indicated generally by the reference numeral 200 is described. In this embodiment, the tray housing is provided in the form of a booklet, but it will be appreciated that the system and method of the invention is applicable for use with different types of tray housings. The tray housing comprises booklet comprising a front page 202, rear page 204 and hinge 206 connecting the front and rear pages allowing the pages to be opened and closed like a book. Each page comprises four rows of seven apertures 208. When the rear page and front page are brought together, the apertures of the front page align in register with the apertures of the rear page. An inner edge 210 of the rear page 204 includes a barcode 212, and an outer edge 212 includes a tab section 214 providing a surface on which an identification sticker may be appended. In this embodiment, the tray housing is formed from stiff cardboard, but other materials may be employed.

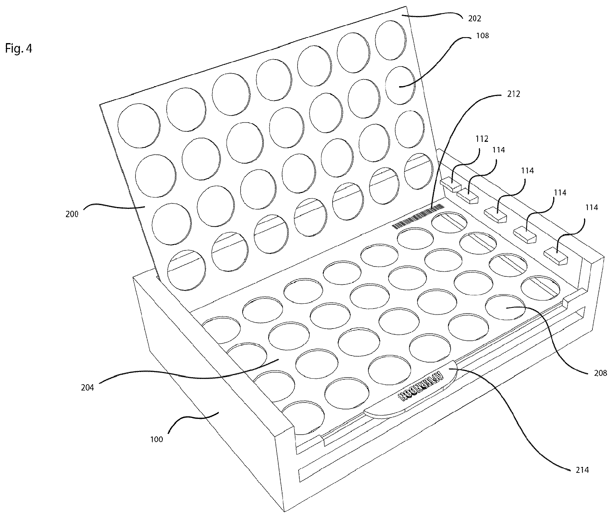

Figure 4 illustrates a tray housing 200 of Figure 3 mounted to a holder 100 of Figures 1 and 2. The booklet is opened, and the rear page 204 is placed into the recess until it nests on the shoulder 108 where it is supported. Once in this position, the barcode 212 is located under the barcode reader 112 and the second barcode readers 114 is disposed over the first aperture 208 in each row of apertures. The tab section 214 projects proud of front wall 102.

Figure 5A to 5D illustrate a drug blister strip 300 comprising a row of seven blisters containers 302. The blister strip is of conventional construction having a frangible top 303 and a dome-shaped crushable base 304 that in use contains a unit dose of a drug. Pressing the base 304 upwardly results in the drug being pushed through the frangible top. The dome-shaped bases 304 are dimensioned to fit snugly in the apertures 108 of the tray housing 100, and the spacing between the dome-shaped bases is such that the blister strip 300 nests within a row of apertures 108 of the tray housing 100. The frangible top 303 of the first blister container 302A bears a blister strip barcode 308 which provides identification of the drug contained within the blister strip.

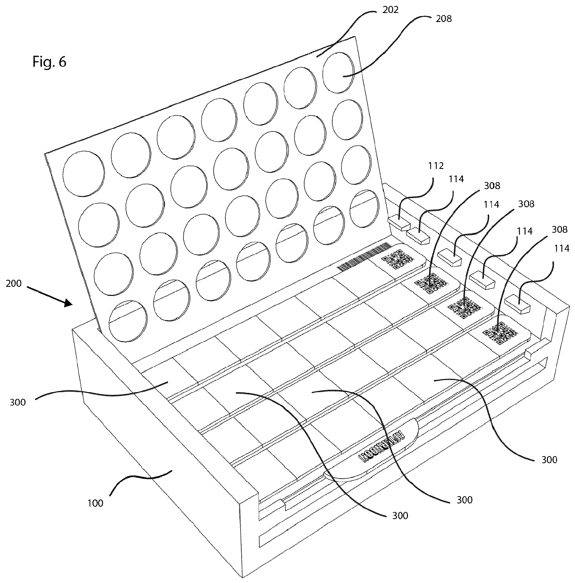

Figure 6 illustrates a tray housing 200 of Figure 3 mounted to a holder 100 of Figures 1 and 2, and with four blister strips 300 mounted to the housing 200 with the dome-shaped bases 304 nested within the apertures of the housing 200 and with the blister strip barcodes 308 disposed underneath the barcode readers 114. Once all of the blister packs have been mounted to the housing 200 and the assembly operator is satisfied that the correct blister strips have been added, the front page 202 of the booklet is closed on to the rear page 204 and secured thereto, and the assembled dispensing tray is removed from the holder 100.

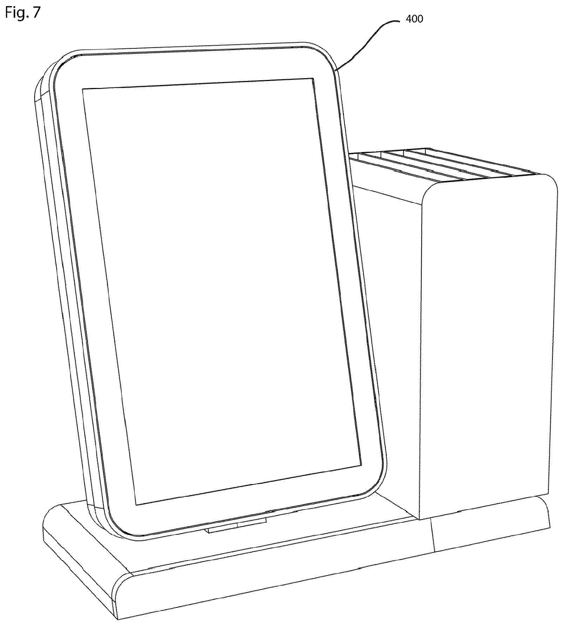

Figure 7 illustrates a graphical display 400 which optionally forms part of the system of the invention. The graphical display is operatively connected to a computing device, and the software forming part of the system of the invention comprises program instructions to cause the graphical display, sensor and processor to work together to display instructions to the assembly operator, for example:
1. Mount a tray housing in the holder
2. Confirm that the barcode of the tray housing has been detected
3. Identify the patient for whom the dispensing tray is being prepared (the identifying information may be anonymised)
4. Identify the first drug blister strip to be mounted to the tray housing
5. Identify the row of the tray housing where the first drug blister strip is to be mounted
6. Confirm that the correct first drug blister strip has been added to the correct row
7. Repeat steps 4 to 6 for each drug blister strip of the prescription.
8. Confirm when all drug blister strips have been added to the tray housing
9. Confirm that a patient-specific data barcode is being printed and is to be applied to the tab of the dispensing tray.

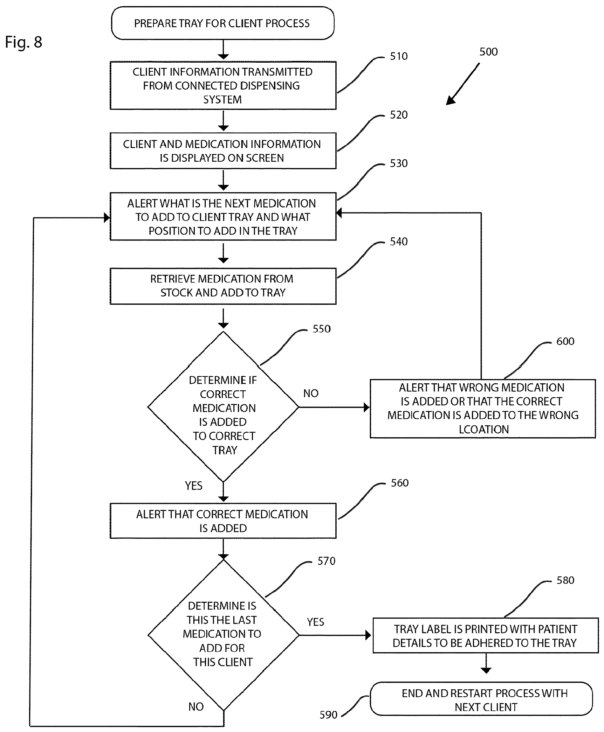

Figure 8 illustrates one embodiment of a method of the invention, indicated generally by the reference numeral 500. Performing a method of the invention may employ a system of the invention including a holder 100 and software that is executable on a computer having a graphical display. The software may be downloadable and may be configured for being executed on a smart phone device. The computer may be wirelessly connected to a patient database that contains details of patients and their prescriptions.

In a first step 510, an assembly operator accesses the patient database using a secure user code and password and retrieves patient data comprising patient identification data and drug prescription data for the patient. The retrieval may be prompted by a computer implemented reminder system. The patient identification data may be anonymised and may refer to the patient as a number. The drug prescription data may include details of daily prescription or, in the case of complex prescriptions, may contain details of a morning prescription. In any event, the information will include details of the drugs making up the prescription and the dosage.

In a second step 520, the patient data is displayed on the screen that can be viewed by the assembly operator.

### PATIENT 12345

| MORNING PRESCRIPTION | |
|---|---|
| SIMVASTATIN | 40 mg |
| DIURIL | 500 mg |
| PLAVIX | 75 mg |

In a third step 530, the graphical display prompts the assembly operator to add the first drug blister strip to a first row of the tray housing in the holder.

In a fourth step 540, the operator retrieves the first drug blister strip from stores and mounts it to the first row of the tray housing.

In a fifth step 550, a second barcode reader reads the barcode of the drug blister strip added to the tray housing in step 540 and relays the barcode information to a processor of the computer, where it is correlated with the patient data to determine if the correct drug blister strip has been added.

If it is determined that the correct drug has been added to the tray housing, then step 560 is performed and a message is displayed on the graphical display that the correct drug blister strip has been added to the tray housing. If it is determined that the incorrect drug has been added to the tray housing, then step 600 is performed and a message is displayed on the graphical display that the incorrect drug blister strip has been added to the tray housing, and the process returns to step 530 where the correct first drug blister strip to be added is displayed on the screen.

In step 570, it is determined if the last drug blister strip is the last drug to be added to the tray housing.

If the answer is YES, a printer prints a label containing the patient data which is dimensioned to be affixed to the tab of the tray housing (step 580). The graphical display will alert the operator that the label is being printed and request the operator to attach the label to the tab of the tray. The graphical display will then ask the operator if they wish to carry out the method of the invention for the next patient (590).

If the answer is NO, steps 530 to 570 are repeated until the final drug blister strip has been added to the tray housing.

In a further step not illustrated in Figure 8, the processor compiles a data file relating to the performed method and including:
- The identity of the patient (including name, date of birth and optionally address)
- The date that the medicament dispensing tray was assembled
- The identity of the assembly operator
- The identity of the tray housing (booklet) employed
- The identity of each of the drug blister strips mounted to the tray housing
- The date the medicament dispensing tray was dispatched to the patient.

The data file is stored in a memory, generally in the patient database, and is retrievable by the pharmacy as and when required. The data file provides evidence of the drugs contained in the medicament dispensing tray, and details of when it was prepared, by whom, and when it was dispatched to the patient.

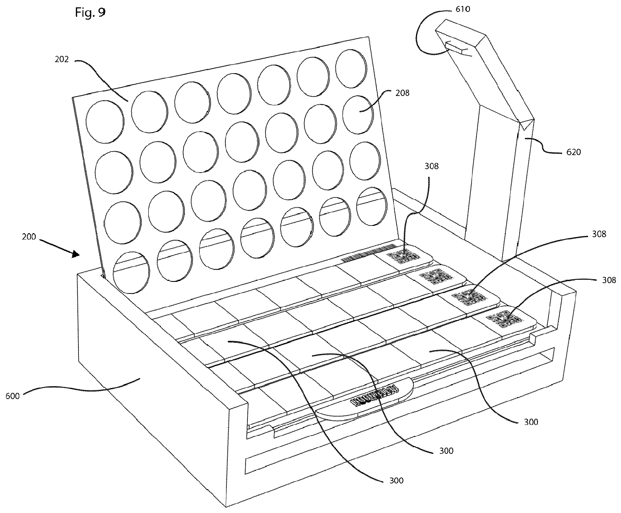

Figure 9 illustrates a tray housing 200 mounted to a tray holder 600 according to an alternative embodiment of the invention and with four blister strips 300 mounted to the housing 200. In this embodiment, the tray holder 600 has a single barcode reader 610 mounted on an arm 620 and configured to read the barcodes of all four blister strips.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. A system to assemble a medicament dispensing tray of the type comprising a tray housing (200) having a machine-readable tray housing identification code (212) and a plurality of drug blister strips (300) mounted to receptacles of the tray housing, each drug blister strip having a machine-readable strip identification code (308), the system comprising:
a device comprising a sensor system configured to detect a tray housing identification code and strip identification codes; and
software executable on a computational device comprising program instructions configured to:
retrieve, by the computational device from a patient database stored on a memory, patient data comprising patient identification data and drug prescription data for the patient;
display the retrieved drug prescription data on a graphical display (400);
detect, by the sensor system a tray housing identification code (212) of a tray housing (200)
detect, by the sensor system, strip identification codes (308) of drug blister strips (300) mounted to the tray housing (100) by the assembly operator;
compare the drug prescription data with the detected strip identification codes;
display a message on the graphical display based on the comparison;
compile a patient-specific data package for the patient comprising the patient identification data, tray identification code, and the plurality of strip identification codes; and
store, by a memory, the patient-specific data package.

2. A system according to Claim 1, in which the software comprises program instructions configured to:
cause the graphical display to display details of a first drug of the drug prescription on the display;
detect with the sensor system when a drug blister strip corresponding to the first drug is mounted to the tray housing;
optionally, display on the graphical display a message indicating that the first drug was successfully added to the tray housing;
display details of a second drug of the drug prescription on the display;
detect with the sensor system when a drug blister strip corresponding to the second drug is mounted to the tray housing; and
optionally, displaying on the graphical display a message indicating that the second drug was successfully added to the tray housing;

3. A system according to Claim 1 or 2, in which the device comprising a sensor system is a mobile communications device with a camera and software for reading machine readable codes.

4. A system according to Claim 1 or 2, comprising a holder (100) to hold the tray housing (200) while drug blister strips (300) are mounted to the tray housing and comprising a holder housing configured to receive the tray housing, and the sensor system.

5. A system according to Claim 4, in which the sensor system comprises a first sensor (112) configured to detect a tray housing identification code of the tray housing received within the holder housing, and one or more second sensors (114) each configured to detect a strip identification code of the drug blister strip mounted to the tray housing received within the housing.

6. A system according to any preceding Claim, in which the processor comprises program instructions configured to cause the graphical display to display an error message when a second sensor detects a strip identification code corresponding to a drug that does not form part of the drug prescription data.

7. A system according to any preceding Claim, in which the processor comprises program instructions configured to cause a printer coupled to the computational device to print the patient-specific data package on to a medium that is attachable to the tray housing.

8. A system according to any preceding Claim, in which the software comprises program instructions configured to cause the graphical display to display for the assembly operator (a) instructions comprising mounting specific drug blister strip to specific receptables of the tray housing and (b) an indication when the instructions have been successfully carried out, in which the software comprises program instructions configured to cause the graphical display to display an indication when all the drug blister strips have been mounted to the tray housing correctly.

9. A system according to any preceding Claim, in which the patient-specific data package for the patient comprises data relating to the batch/lot of the drugs contained in one or more of the drug blister strips of the medicament dispensing tray.

10. A system according to any preceding Claim, in which the patient-specific data package for the patient comprises information relating to how the drugs in the drug blister strips of the medicament dispensing tray relates back to an original prescription.

11. A system according to any preceding Claim, in which the patient-specific data package for the patient comprises the scheduled and/or actual delivery date of the medicament dispensing tray to the patient.

12. A system according to any preceding Claim, in which the tray housing comprises a booklet comprising a front page and a back page, the front page having an array of apertures comprising at least two rows of seven apertures, in which the back page comprises a corresponding array of apertures configured to register with the apertures of the front page when the booklet is closed.

13. A system according to Claim 12, in which a plurality of drug blister strips includes a first blister strip containing a first drug and a second blister strip containing a second drug, wherein each blister strip comprises a row of seven blisters corresponding to the plurality of apertures in each row of apertures allowing the blister strip to nest snugly in a row of apertures of the front or back page of the booklet.

14. A method of assembling a medicament dispensing tray comprising a tray housing having a machine-readable tray housing identification code and a plurality of drug blister strips mounted to receptacles of the tray housing, each strip having a machine-readable strip identification code, the method employing a holder to hold the tray housing while drug blister strips are mounted to the tray housing and comprising a holder housing configured to receive the tray housing, a first sensor configured to detect a tray housing identification code of the tray housing received within the holder housing, and a plurality of second sensors each configured to detect a strip identification code of the drug blister strip mounted to the tray housing received within the housing, the method comprising the steps of:
retrieving from a patient database stored on a memory, patient data comprising patient identification data and drug prescription data for the patient;
displaying the retrieved drug prescription data on a graphical display;
placing a tray housing in the holder housing;
detecting, by the first sensor, a tray identification code of the tray housing placed within the holder;
mounting a plurality of drug blister strips to receptacles of the tray housing according to the retrieved drug prescription data displayed on the graphical display;
detecting, by the plurality of second sensors, strip identification codes of drug blister strips mounted to the dispensing tray;
comparing the retrieved drug prescription data with the detected strip identification codes;
displaying a message on the graphical display based on the comparison;
compiling a patient-specific data package for the patient comprising the patient identification data, tray identification code, and the plurality of strip identification codes; and
storing, by the memory, the patient-specific data package.

15. A method according to Claim 14, in which the method comprises the steps of:
displaying details of a first drug of the retrieved drug prescription on the graphical display;
mounting a drug blister strip containing the first drug to a receptable of the tray housing;
detecting with a second sensor when the drug blister strip containing the first drug is mounted to the tray housing;
optionally, displaying on the graphical display a message indicating that the first drug was successfully added to the tray housing;
displaying details of a second drug of the drug prescription on the graphical display; and
mounting a drug blister strip containing the second drug to a receptable of the tray housing; and
detecting with a second sensor when the drug blister strip containing the second drug is mounted to the tray housing;
optionally, displaying on the graphical display a message indicating that the second drug was successfully added to the tray housing;
